# EUROPEAN PATENT APPLICATION

(11) **EP 4 674 450 A1**
(43) Date of publication of application: **07.01.2026**
(21) Application number: 24860399.5
(22) Date of filing: 28.08.2024
(51) Int. Cl.: A61M 5/168, A61M 5/38, A61M 5/40, A61M 5/165

(54) **REGULATOR-INTEGRATED DRIP CHAMBER HAVING LOCKING DEVICE AND INFUSION SET COMPRISING SAME**

(30) Priority: 31.08.2023 KR 20230115196
(71) Applicant: Medicpro Korea Co., Ltd., Chungcheongnam-do 32710 (KR)
(72) Inventor: YOON, Sung-min, Seoul 03947 (KR); YOON, Boo-yong, Seoul 03497 (KR); YOON, Sung-hwan, Seoul 03947 (KR)
(74) Representative: Isarpatent
(86) International application number: PCT/KR2024/012832
(87) International publication number: WO 2025/048479

(57) **Abstract**

The present invention relates to a regulator-integrated drip chamber having a locking device and an infusion set comprising same and, more specifically, to a regulator-integrated drip chamber having a locking device, the drip chamber being connected to an infusion bag such that the infusion solution in the infusion bag is supplied thereto. The drip chamber comprises: a body unit connected to an infusion bag such that the infusion stored in the infusion bag is supplied therein in a waterdrop type, the body unit having a lower end portion provided with a discharge hole and connected to a supply hose; a regulator integrally coupled to one side of the lower portion of the body unit so as to adjust the flow rate of the infusion solution supplied from the body unit to the supply hose; and a filter member provided on one side of the inside of the lower end of the body unit. The regulator has a top body unit, a bottom body unit, a gasket interposed between the top body unit and the bottom body unit, and a locking device for limiting the rotation of the bottom body unit or releasing same.

## Description

### TECHNICAL FIELD

The present invention relates to a regulator-integrated drip chamber with a locking device, and more particularly, to an infusion set including the same.

### BACKGROUND ART

An infusion set is a type of medical device used to inject infusion fluid into the human body for medical purposes. A typical infusion set includes an infusion bag, a drip chamber that drains the infusion fluid from the infusion bag, a supply hose (tube) connected to the drip chamber, an infusion regulator for controlling the amount of infusion fluid flowing through the supply hose, and a catheter inserted into the patient's vein.

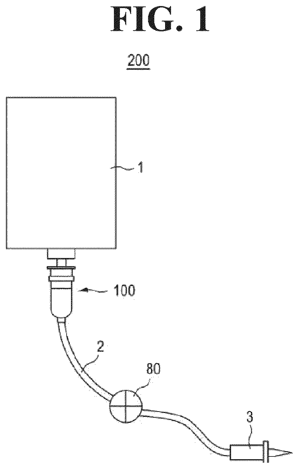

FIG. 1 illustrates a schematic view of a conventional infusion set.

The drip chamber includes: a body portion that stores a certain amount of infusion fluid; an inlet through which the infusion fluid flows into the drip chamber via a spike inserted into the infusion bag; and an outlet that discharges the infusion fluid stored in the drip chamber into the supply hose.

When infusion is administered to a patient using such a conventional infusion set, the infusion bag must be replaced immediately after all the fluid is administered, or the infusion must be stopped and the catheter removed from the patient's vein.

If the infusion set is left unattended after the infusion fluid is depleted, air may enter the patient's vein through the supply hose, or blood may flow back through the hose due to a pressure difference-posing a serious risk.

Therefore, in conventional infusion sets, medical personnel or caregivers must frequently check the remaining amount of infusion fluid in the infusion bag or drip chamber, which is inconvenient.

To address this problem, Korean Patent No. 10-1027081 (issued March 29, 2011) discloses a backflow prevention device in which a buoyant ball with a certain weight and size floats on the infusion fluid inside the drip chamber, and blocks the outlet when the liquid is fully drained.

Also, Korean Patent No. 10-0792805 (issued January 2, 2008) discloses a passive liquid level sensing device that includes: a magnetic float that floats based on the fluid level in the drip chamber; at least two magnetic proximity switches arranged at certain intervals outside the drip chamber and output ON/OFF signals based on the float's position; and an interface connector that electrically connects the signal output terminal of the magnetic proximity switches to an emergency call system or alarm system installed in a hospital.

However, the floating members in these conventional technologies are structured in a way that makes it difficult to accurately seal the discharge hole of the drip chamber, as they merely float on the fluid.

Moreover, these conventional technologies only detect when the infusion supply is exhausted from the bag, and do not provide means to precisely control the amount of infusion administered to the patient according to a preset supplied amount.

### DISCLOSURE OF INVENTION

### TECHNICAL PROBLEM

Accordingly, the present invention has been devised to solve the aforementioned problems. According to embodiments of the present invention, a regulator for adjusting the infusion amount is integrated with the drip chamber, rather than being provided on one side of the supply hose. This allows for a more compact infusion chamber design and facilitates assembly and arrangement of the infusion set. One objective of the present invention is to provide a regulator-integrated drip chamber with a locking device and an infusion set including the same.

According to another embodiment of the present invention, the regulator is equipped with a locking device, which prevents malfunction due to accidental operation by a patient, caregiver, or third party during infusion. Another obj ective of the present invention is to provide a regulator-integrated drip chamber with a locking device and an infusion set including the same.

According to yet another embodiment of the present invention, a flow rate sensor including an optical sensor or the like is installed in the drip chamber to detect the amount and rate of infusion, and to control the flow control unit so that infusion is performed at a preset supplied amount and speed. Yet another objective of the present invention is to provide a regulator-integrated drip chamber with a locking device and an infusion set including the same.

According to still another embodiment of the present invention, the drip chamber includes a liquid level sensor to determine the residual amount of infusion fluid between the drip chamber and the catheter. The infusion amount supplied from the infusion bag and the residual amount can be used to monitor the actual infused amount into the patient and ensure that the total supplied amount corresponds to the preset supplied amount. Still another objective of the present invention is to provide a regulator-integrated drip chamber with a locking device and an infusion set including the same.

Meanwhile, the technical objectives of the present invention are not limited to those described above. Other technical objectives not mentioned herein will be clearly understood by those skilled in the art from the following description of the invention.

### TECHNICAL SOLUTION

According to a first aspect of the present invention, there is provided a drip chamber connected to an infusion bag to receive an infusion fluid from the infusion bag, comprising: a body portion having a lower end portion that is configured to be connected to a supply hose and forms a discharge hole, such that the infusion fluid stored in the infusion bag is supplied into the body portion in the form of droplets; a regulator integrally coupled to one side of a lower portion of the body portion and configured to control a flow rate of the infusion fluid supplied from the body portion to the supply hose; and a filter member provided at one side inside the lower portion of the body portion, wherein the regulator comprises: an upper body part; a lower body part; a gasket interposed between the upper body part and the lower body part; and a locking device configured to restrict or release the rotation of the lower body part.

The upper body part may be integrally coupled to one side of the lower portion of the body portion, and has a discharge port formed at one side of a bottom surface thereof and a central rotation shaft protruding downward from the center of the bottom surface, and the lower body part may have a rotation shaft receiving groove-forming part into which the central rotation shaft is inserted, and a supply port formed at one side of an upper surface thereof. The regulator-integrated drip chamber may be configured to adjust the flow rate of the infusion fluid as the lower body part rotates around the central rotation shaft.

An insertion groove may be formed at a lower end of the central rotation shaft. The locking device may comprise: a locking body disposed between the central rotation shaft and the rotation shaft receiving groove-forming part, the locking body forming a lower end hole and internal threads on an inner surface thereof; a central member disposed in the lower end hole, the central member including a locking end having external threads on an outer surface thereof that are screw-engaged with the internal threads; and a tightening knob configured to rotate the central member, wherein in a locking mode, an upper end of the central member is configured to be forcibly fitted into the insertion groove by the operation of the tightening knob to restrict rotation of the lower body part.

Threads may be formed on an outer surface of a lower portion of the central rotation shaft, and the rotation shaft receiving groove-forming part may have a tubular shape with slits that are spaced apart in the circumferential direction, wherein the central rotation shaft is inserted and coupled through the rotation shaft receiving groove-forming part such that the threads of the central rotation shaft are exposed at a lower side thereof. The locking device may comprise: a locking body screw-coupled to the lower portion of the central rotation shaft via internal threads of the locking body; and a tightening knob connected to a lower end of the locking body and configured to rotate the locking body, wherein in a locking mode, the tightening knob is configured to press the rotation shaft receiving groove-forming part to restrict the rotation of the lower body part.

The drip chamber may comprise: a flow rate sensor provided at one side of the body portion for detecting infusion droplets passing through the body portion and measuring the supplied infusion amount; a floating member that floats on the infusion fluid stored in the body portion and closes the discharge hole when the infusion supply is completed; and a flow rate calculator configured to calculate the flow rate and total supplied amount based on the droplet interval or the number of droplets supplied during a specific period, based on values transmitted from the flow rate sensor, wherein the flow rate sensor comprises at least one of an optical sensor, infrared sensor, or laser sensor for detecting the supplied infusion droplets.

According to a second aspect of the present invention, there is provided an infusion set comprising: the infusion bag; the drip chamber according to the first aspect; the supply hose; a catheter; a flow control unit provided at one side of the supply hose and configured to regulate the flow rate of the infusion fluid supplied; and a management server configured to calculate the flow rate and a total supplied amount of the infusion fluid based on an interval between infusion droplets or the number of droplets supplied during a certain period, using measurement data received from a flow rate sensor.

The infusion set may comprise: a setting module configured to set a supplied amount and the flow rate of the infusion fluid; and a controller configured to control the flow control unit based on the measurement data and the preset flow rate and to stop the infusion when the total supplied amount reaches the preset supplied amount.

The infusion set may also comprise: a liquid level sensor configured to measure a level of the infusion fluid in the drip chamber; and a residual infusion fluid calculator configured to compute the remaining amount of infusion fluid from the drip chamber to a tip of the catheter based on the level of the infusion fluid, wherein the controller is configured to control the flow control unit to stop the infusion when a value obtained by subtracting the remaining amount of infusion fluid from the total supplied amount reaches the preset supplied amount.

### ADVANTAGEOUS EFFECTS

According to embodiments of a regulator-integrated drip chamber with a locking device and an infusion set including the same, since the regulator for adjusting the amount of infusion is integrated with the drip chamber instead of being located on the side of the supply hose, a more compact infusion chamber can be configured, and convenience in assembling and arranging the infusion set can be achieved.

According to embodiments of a regulator-integrated drip chamber with a locking device and an infusion set including the same, since the regulator is provided with a locking device, malfunction due to unintended operation by a patient, caregiver, or others during infusion can be prevented.

According to embodiments of a regulator-integrated drip chamber with a locking device and an infusion set including the same, a flow rate sensor comprising an optical sensor or the like is installed in the drip chamber to detect the amount and rate of the supplied infusion, and the flow control unit can be controlled so that infusion is supplied in accordance with the preset supplied amount and rate.

According to embodiments of a regulator-integrated drip chamber with a locking device and an infusion set including the same, by including a liquid level sensor, the residual infusion volume from the drip chamber to the catheter can be determined. Based on the supplied infusion volume from the infusion bag and the residual volume, the actual amount of infusion delivered to the patient can be monitored, and the system can be controlled so that the actual infused amount corresponds to the preset supplied amount.

Meanwhile, the effects obtainable from the present invention are not limited to those mentioned above, and other effects not mentioned will be clearly understood by those skilled in the art from the following description of the invention.

### BRIEF DESCRIPTION OF THE DRAWINGS

The drawings attached to this specification illustrate preferred embodiments of the present invention and, together with the detailed description of the invention, serve to further clarify the technical features of the invention. However, the present invention is not to be construed as being limited to the matters described in the drawings.
FIG. 1 is a schematic view of a conventional infusion set.
FIG. 2 is a front view of a regulator-integrated drip chamber (chamber cover type) according to an embodiment of the present invention.
FIG. 3 is a front view of a regulator-integrated drip chamber (spike type) according to an embodiment of the present invention.
FIG. 4 is a front view of a regulator-integrated drip chamber (chamber cover type) with a flow rate sensor according to an embodiment of the present invention.
FIGS. 5 and 6 are front views of a regulator-integrated drip chamber (chamber cover type) with a flow rate sensor and a floating member according to an embodiment of the present invention.
FIG. 7 is a front view of a regulator-integrated drip chamber (chamber cover type) with a flow rate sensor, a liquid level sensor, and a floating member according to an embodiment of the present invention.
FIG. 8 is a sectional view of a regulator-integrated drip chamber with a locking device according to a first embodiment of the present invention.
FIG. 9 is a sectional view of FIG. 9 with the lower body part removed.
FIG. 10 is an enlarged sectional view of the locking device part shown in FIG. 8.
FIG. 11 shows sectional and plan views of the locking body, central member, and locking lever of the locking device according to the first embodiment of the present invention.
FIG. 12 is a sectional view of the locking device in the unlocking mode according to the first embodiment of the present invention.
FIG. 13 is a sectional view of the locking device in the locking mode according to the first embodiment of the present invention.
FIGS. 14 and 15 are a perspective view and a front view of the regulator according to a second embodiment of the present invention.
FIGS. 20 and 21 are a perspective view and a front view of the upper body part of the regulator according to the second embodiment of the present invention.
FIGS. 22 and 23 are a perspective view and a plan view of the gasket of the regulator according to the second embodiment of the present invention.
FIGS. 24 to 25 are a perspective view, front view, and bottom view of the lower body part of the regulator according to the second embodiment of the present invention.
FIGS. 27 and 28 are a perspective view and a plan view of the locking device according to the second embodiment of the present invention.
FIG. 29 is a partial sectional view of the locking device in the unlocking mode according to the second embodiment of the present invention.
FIG. 30 is a partial sectional view of the locking device in the locking mode according to the second embodiment of the present invention.
FIG. 31 is a block diagram of an infusion set including the regulator-integrated drip chamber according to an embodiment of the present invention.
FIG. 32 is a block diagram showing the signal flow in the controller according to an embodiment of the present invention.

### BEST MODE FOR INVENTION

Hereinafter, the structure and function of a regulator-integrated drip chamber according to an embodiment of the present invention will be described.

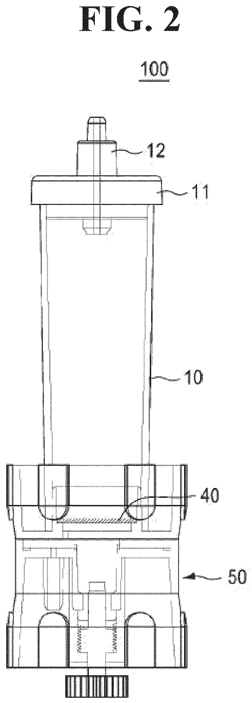

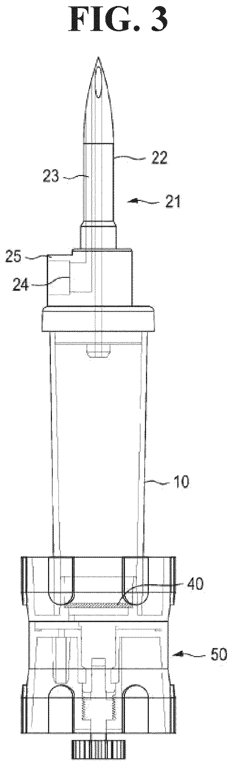

First, FIG. 2 is a front view of a regulator-integrated drip chamber (chamber cover type) according to an embodiment of the present invention. FIG. 3 is a front view of a regulator-integrated drip chamber (spike type) according to an embodiment of the present invention.

The drip chamber according to an embodiment of the present invention is connected to an infusion bag to receive infusion fluid. As shown in FIG. 3, the spike type may include an infusion port 22, an air port 23, an air filter 24, and a vent cap 25. As shown in FIG. 2, the chamber cover type may include a chamber cover 11 and a connection connector 12 connected to the infusion bag. The invention can be implemented in either of these configurations, though the following description will focus on the chamber cover type by way of example. However, the scope of the invention is not limited to that type.

The body portion 10 of the drip chamber 100 is connected to the infusion bag 1, and the infusion fluid stored in the infusion bag is supplied into the body portion in the form of droplets. The body portion has a lower end portion having a discharge hole and connected to a supply hose 2.

In this embodiment, the drip chamber 100 includes a regulator 50 that is integrally coupled to one side of the lower portion of the body portion 10, and is configured to adjust the flow rate of the infusion fluid supplied from the body portion 10 to the supply hose 2.

A filter member 40 is also provided on one side inside a lower portion of the body portion 10 to filter out foreign substances and the like. The filter member 40 may be composed of a hydrophilic membrane so as to prevent gas (air) from entering the supply hose.

The regulator 50 according to this embodiment of the present invention may include an upper body part 51 and a lower body part 55 that can be rotated relative to the upper body part 51.

The upper body part 51 is integrally coupled to one side of a lower portion of the body portion 10 of the drip chamber 100, and includes a discharge port 52 formed on one side of a bottom surface thereof, and a central rotation shaft 53 protruding downward from the center of the bottom surface. In addition, a filter member 40 may be installed inside the upper body part 51 to ensure patient safety by preventing the entry of foreign matter and the injection of air.

The lower body part 55 includes a rotation shaft receiving groove-forming part 57 into which the central rotation shaft 53 is inserted, and a supply port 56 formed on one side of the upper surface. Thus, as the lower body part 55 rotates about the central rotation shaft 53, the infusion flow rate can be adjusted.

An insertion groove is formed on one side of the outer surface of the lower end of the central rotation shaft 53, and a locking step 58 is formed on the outer lower surface of a projecting tube that forms the rotation shaft receiving groove of the lower body part 55. Therefore, the projecting tube connected to the lower body part 55 supports the center of the central rotation shaft 53 of the upper body part 51, functioning as a locking mechanism that prevents the separation of the upper body part 51 and the lower body part 55.

A gasket 59 made of silicone is placed in the space between the contact surfaces of the upper body part 51 and the lower body part 55, thereby allowing the upper body part 51 and the lower body part 55 to rotate smoothly along the central axis and preventing leakage.

As will be described in detail later, the regulator 50 according to the embodiment of the present invention may further include a locking device 60 that restricts the rotation of the lower body part 55.

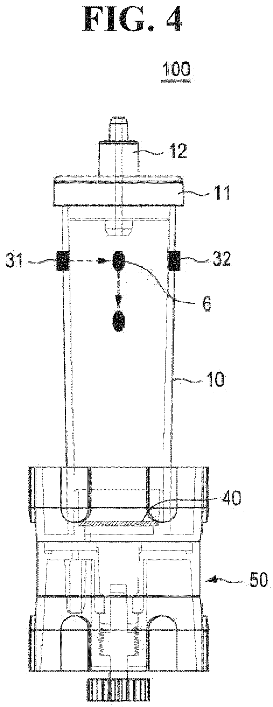

FIG. 4 is a front view of a regulator-integrated drip chamber (chamber cover type) with a flow rate sensor according to an embodiment of the present invention.

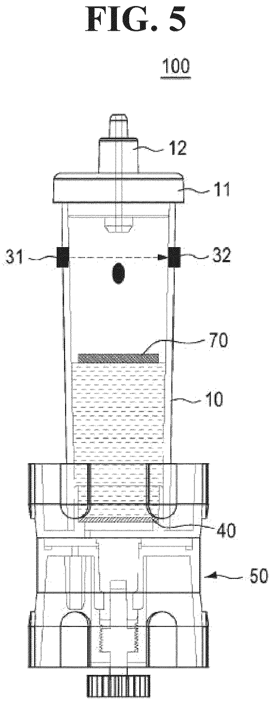

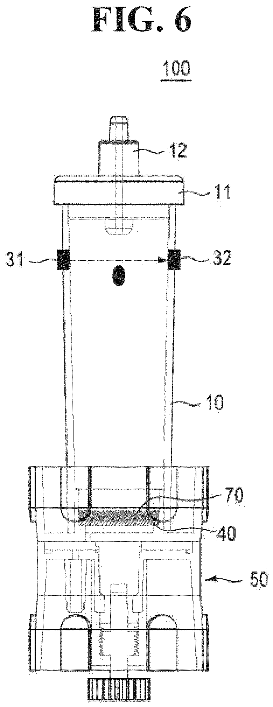

FIGS. 5 and 6 are front views of a regulator-integrated drip chamber (chamber cover type) with both a flow rate sensor and a floating member according to an embodiment of the present invention.

According to this embodiment, the drip chamber 100 includes a flow rate sensor 30 provided on one side of the body portion 10, which detects infusion droplets passing through the body portion 10 and measures the supplied volume of infusion.

A floating member 70 is configured to float on the infusion fluid stored in the body portion 10, and when the infusion supply is completed, it closes the discharge hole 62.

The flow rate sensor 30 may be composed of an optical sensor, infrared sensor, or laser sensor, and may include a light emitter 31 for emitting light and a light receiver 32 for receiving reflected light. These sensors detect falling infusion droplets, and a flow rate calculator 33 receives the measurement values and calculates the flow rate and total supplied amount based on the interval of droplets or the number of droplets supplied during a certain time.

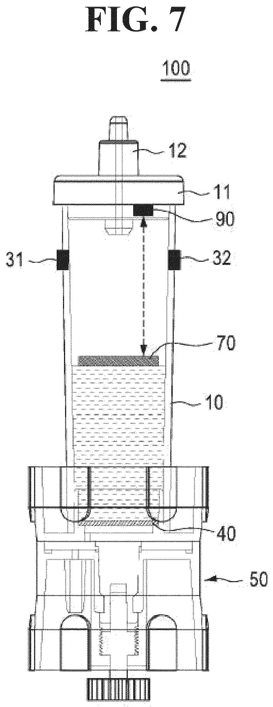

FIG. 7 is a front view of a regulator-integrated drip chamber (chamber cover type) with a flow rate sensor, liquid level sensor, and floating member according to an embodiment of the present invention.

As shown in FIG. 7, the drip chamber 100 may further include a liquid level sensor 90 for measuring the fluid level inside the drip chamber.

In this configuration, the floating member 70 may serve as a reflector. Therefore, the liquid level sensor may be configured to measure the fluid level in the drip chamber 100 based on the position of the floating member 70.

Alert data may be transmitted if the fluid level drops below a preset threshold.

As will be described later, a residual infusion fluid calculator 91 may also be included in the management server 110, configured to calculate the residual amount of infusion fluid from the drip chamber 100 through the supply hose 2 to the tip of the catheter 3 based on the liquid level.

Accordingly, the controller 120 subtracts the residual infusion amount (calculated by the residual infusion fluid calculator 91) from the total supplied amount (calculated by the flow rate calculator 33), and controls the flow control unit 80 to stop infusion if the result reaches the preset supplied amount.

That is, when information on the length and inner diameter of the supply hose 2 and the residual infusion volume based on the size of the supply hose is input, and the fluid level in the drip chamber 100 is obtained from the liquid level sensor 90, the infusion volume remaining from the catheter to the drip chamber can be calculated in real time.

By subtracting this residual infusion volume from the total amount supplied from the infusion bag 1 to the drip chamber 100, the actual volume delivered to the patient can be calculated. Then, based on this actual delivered volume, the controller 120 is configured to stop infusion through the flow control unit 80 once the actual delivered volume matches the preset supplied amount.

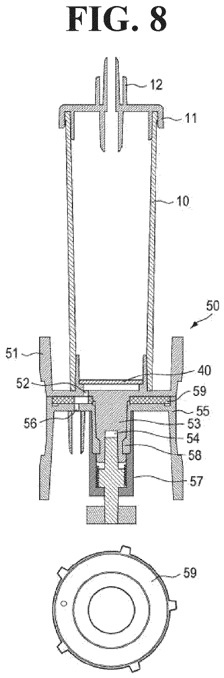

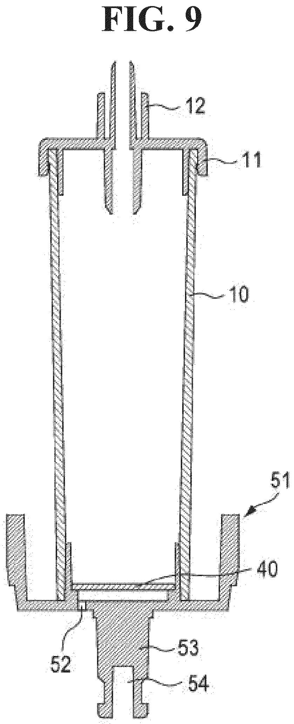

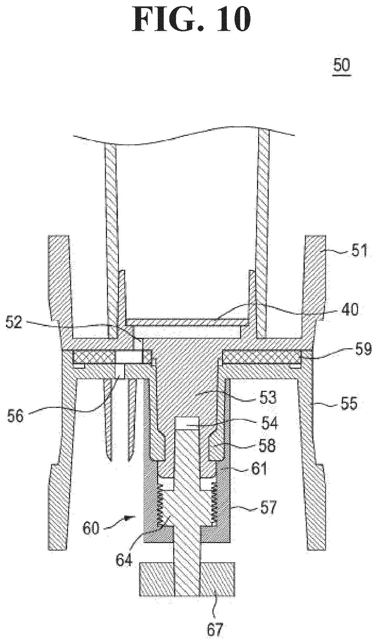

FIG. 8 illustrates a sectional view of a regulator-integrated drip chamber with a locking device according to the first embodiment of the present invention. FIG. 9 illustrates a sectional view of FIG. 9 in which the lower body part is excluded. FIG. 10 illustrates an enlarged sectional view of the locking device portion shown in FIG. 8.

FIG. 1 1 illustrates a sectional view of a locking body, a sectional view of a central member of a locking member, a sectional view of a lever of the locking member, and a plan view according to the first embodiment of the present invention.

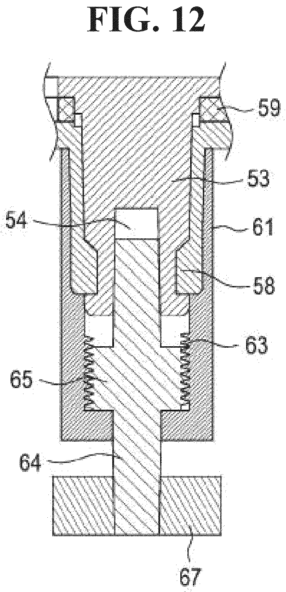

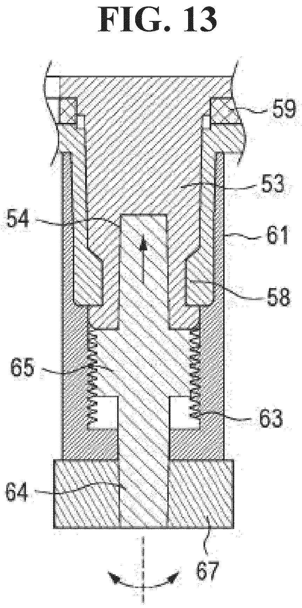

FIG. 12 illustrates a sectional view of the locking device in an unlocking mode according to the first embodiment of the present invention, and FIG. 13 illustrates a sectional view of the locking device in a locking mode according to the embodiment of the present invention.

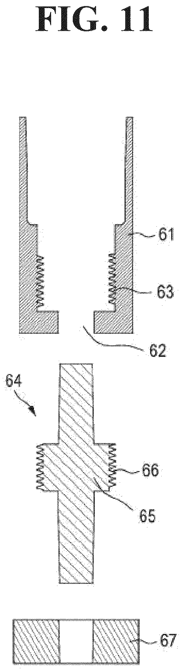

As illustrated in FIGS. 8, 9, 10, 11, 12, and 13, the regulator 50 may include a locking device 60 configured to restrict rotation of the lower body part 55.

The locking device according to the first embodiment may include a locking body 61 having internal threads 63, and a central member 64 connected to a lever and including a locking end 65 that is threadably engaged with the internal threads 63.

First, an insertion groove 54 is formed on the lower end of the central rotation shaft 53 of the upper body part 51 of the regulator 50.

The locking body 61 is interposed between the central rotation shaft 53 and the rotation shaft receiving groove-forming part 57, and forms a lower end hole 62 and internal threads 63 on its inner surface.

The central member 64 is disposed in the lower end hole 62 and is configured to have a locking end 65 forming external threads 66 on its outer surface, which are threadably engaged with the internal threads 63. The central member 64 is connected to a lever 67 for rotating the central member.

Accordingly, in the unlocking mode, as illustrated in FIG. 12, the upper end of the central member 64 is spaced apart from an upper surface of the insertion groove 54 of the central rotation shaft 53, so that the lower body part 55 can rotate without restriction.

In the locking mode, as illustrated in FIG. 13, when the lever 67 is operated to rotate the central member 64, the central member 64 moves upward by the thread engagement. As a result, the upper end of the central member 64 is forcibly fitted into the insertion groove 54, thereby restricting the rotation of the lower body part 55.

### MODE FOR INVENTION

Hereinafter, the structure and function of the regulator and the locking device according to the second embodiment of the present invention will be described.

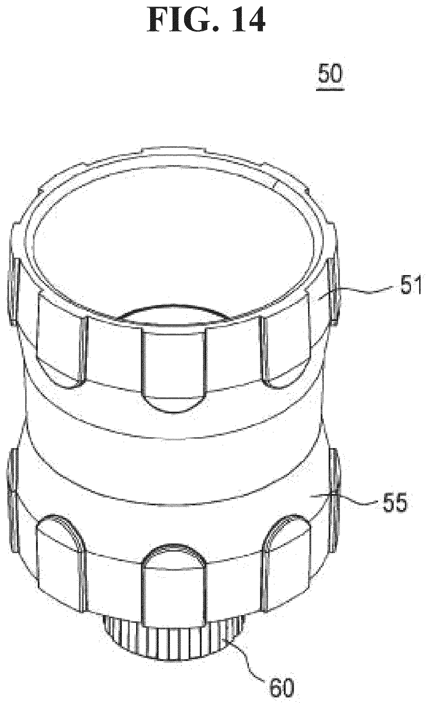

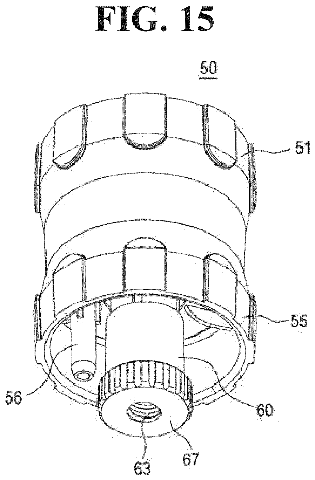

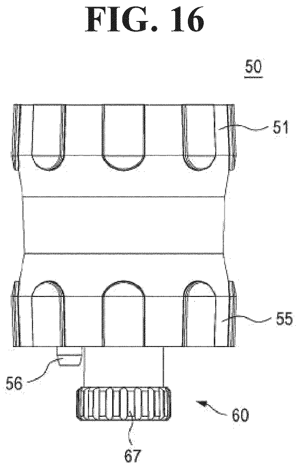

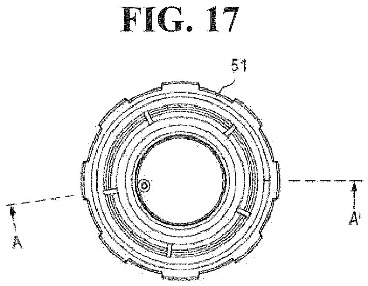

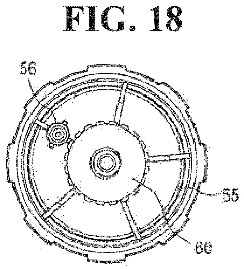

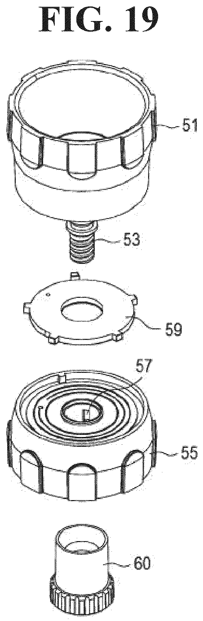

FIGS. 14 and 15 are a perspective view and a front view of the regulator according to the second embodiment of the present invention.

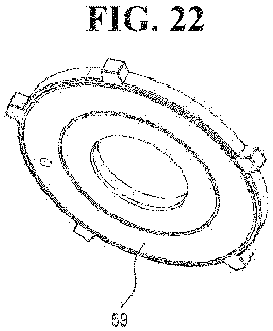

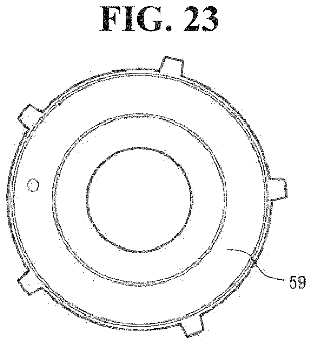

he regulator of the second embodiment has a configuration in which an upper body part 51 and a lower body part 55 are assembled together, with a gasket 59 interposed therebetween. A gasket 59, as shown in FIGS. 22 and 23, is interposed between the upper body part 51 and the lower body part 55 to complete the assembly.

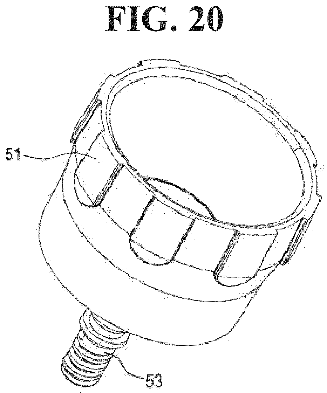

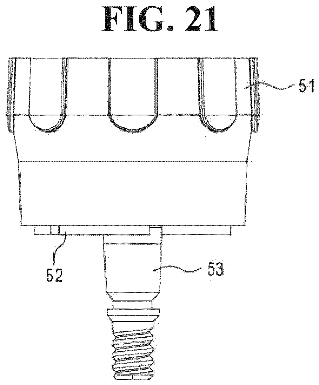

FIGS. 20 and 21 show a perspective view and a front view of the upper body part of the regulator.

FIGS. 22 and 23 show a perspective view and a plan view of the gasket.

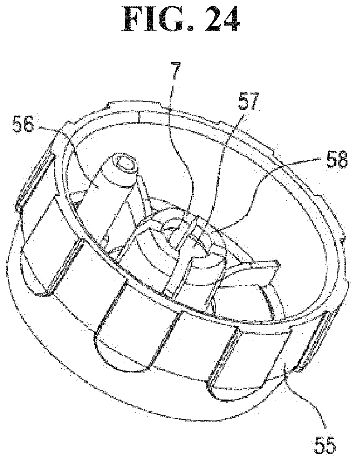

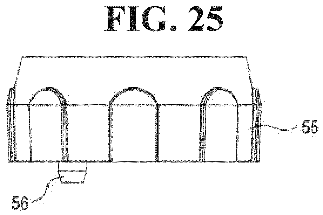

FIGS. 24 and 25 show a perspective view, a front view, and a bottom view of the lower body part.

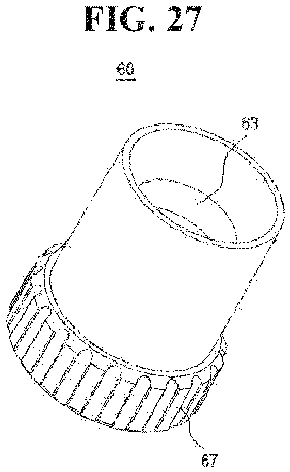

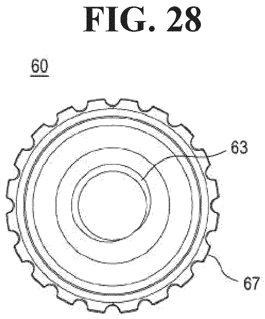

FIGS. 27 and 28 show a perspective view and a plan view of the locking device.

As shown in FIGS. 20 and 21, the upper body part 51 includes a discharge port 52, and a central rotation shaft 53 protruding downward from the bottom surface.

Threads are formed on the outer surface of the lower portion of the central rotation shaft 53, and these threads are configured to engage with internal threads 63 of the locking device 60 described later.

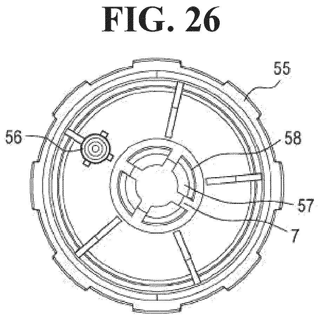

As shown in FIGS. 24 to 26, the lower body part 55 is assembled with the upper body part 51 with the gasket interposed. In the unlocking mode, the lower body part 55 can rotate to open or close the discharge port 52, while in the locking mode, the rotation is restricted.

The lower body part 55 includes a supply port 56, and a rotation shaft receiving groove-forming part 57 through which the central rotation shaft 53 of the upper body part 51 is inserted.

When the upper body part 51 and the lower body part 55 are assembled, the central rotation shaft 53 passes through the rotation shaft receiving groove-forming part 57, and the threads on the shaft are exposed at the bottom. The locking device is then threadably coupled to the exposed portion of the shaft.

Additionally, the rotation shaft receiving groove-forming part 57 includes a plurality of longitudinal slits that are spaced apart in the circumferential direction. Thus, as described below, when the locking device is rotated and moved upward in the locking mode, the spacing between the slits is reduced, compressing the receiving groove-forming part 57 and restricting the rotation of the lower body part 55.

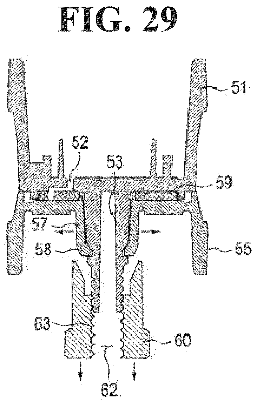

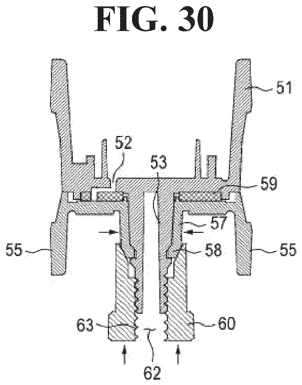

More specifically, FIG. 29 is a partial sectional view of the locking device in the unlocking mode, and FIG. 30 is a partial sectional view of the locking device in the locking mode, both according to the second embodiment of the present invention.

As shown in FIGS. 29 and 30, in the state where the upper body part 51 and the lower body part 52 are assembled, the central rotation shaft 51 with external threads is inserted through the rotation shaft receiving groove-forming part 57. In this state, the threads are exposed at the bottom, and the locking device 60 is engaged by being screwed onto the threads.

As shown in FIG. 29, in the unlocking mode, the locking projection 58 of the rotation shaft receiving groove-forming part 57 and the central rotation shaft 51 are spaced apart from each other, so the lower body part 55 is not constrained and can rotate around the central rotation shaft 51.

As shown in FIG. 30, in the locking mode, when the tightening knob 67 of the locking device 60 is rotated by a user or an automatic mechanism, the top end of the locking device 60 comes into contact with the locking projection 58 of the rotation shaft receiving groove-forming part 57. With further rotation, the receiving groove 57 is pressed and compressed. The groove has multiple slits 7 formed in a circumferential arrangement, and as the compression increases, it becomes forcibly engaged with the central rotation shaft 53. In this state, the rotation of the lower body part 55 is restricted.

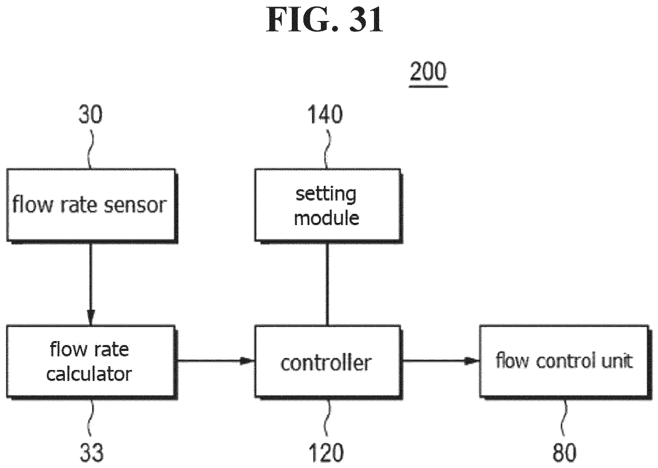

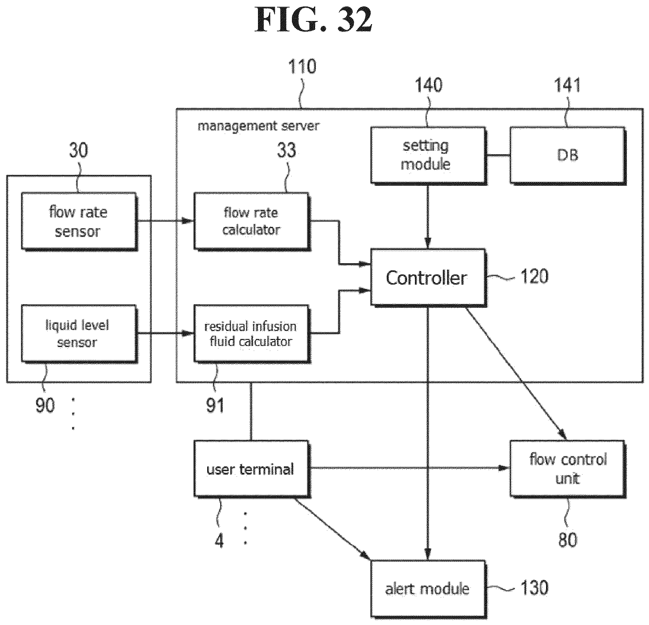

FIG. 31 is a block diagram of an infusion set including a regulator-integrated drip chamber with a locking device according to an embodiment of the present invention. FIG. 32 is a block diagram illustrating the signal flow of the controller according to an embodiment of the present invention.

According to the embodiment shown, the infusion set 200 includes a flow control unit 80 provided on one side of the supply hose 2 for regulating the infusion flow rate.

The management server 110 receives measurement data from the flow rate sensor 30 and includes a flow rate calculator 33 configured to calculate the flow rate and total infusion amount based on the interval of infusion droplets or the number of droplets supplied during a specific period. A setting module is configured to set the infusion amount and flow rate, and the controller controls the flow control unit 80 based on the measured data from the flow rate calculator 33 and the preset flow rate from the setting module 140.

The controller 120 is also configured to stop infusion once the total infusion amount reaches the preset supplied amount by controlling the flow control unit 80.

As shown in FIG. 7, the drip chamber 100 may further include a liquid level sensor 90 to measure the fluid level inside the chamber. Additionally, the management server 110 may include a residual infusion fluid calculator 91 configured to calculate the remaining infusion amount from the drip chamber 100, through the supply hose 2, to the tip of the catheter 3, based on the measured fluid level.

Accordingly, the controller 120 calculates the value obtained by subtracting the residual infusion amount (calculated by the residual infusion fluid calculator 91) from the total supplied amount (calculated by the flow rate calculator 33) and stops infusion when this value reaches the preset supplied amount.

In other words, when information on the length and inner diameter of the supply hose 2 is provided and the residual amount of the infusion fluid based on the hose size is inputted, if the liquid level sensor 90 detects the fluid level in the drip chamber 100, the system can compute in real time the volume of infusion remaining from the catheter to the drip chamber.

By subtracting this residual amount from the total amount supplied from the infusion bag 1 to the drip chamber 100, the actual amount of infusion delivered to the patient can be calculated.
Then, if the actual infusion amount reaches the preset supplied amount, the controller 120 controls the flow control unit 80 to stop the infusion.

## Claims

1. A regulator-integrated drip chamber connected to an infusion bag to receive an infusion fluid from the infusion bag, comprising:
a body portion having a lower end portion that is configured to be connected to a supply hose and forms a discharge hole, such that the infusion fluid stored in the infusion bag is supplied into the body portion in the form of droplets;
a regulator integrally coupled to one side of a lower portion of the body portion and configured to control a flow rate of the infusion fluid supplied from the body portion to the supply hose; and
a filter member provided at one side inside the lower portion of the body portion,
wherein the regulator comprises:
an upper body part;
a lower body part;
a gasket interposed between the upper body part and the lower body part; and
a locking device configured to restrict or release the rotation of the lower body part.

2. The regulator-integrated drip chamber of claim 1,
wherein the upper body part is integrally coupled to one side of the lower portion of the body portion, and has a discharge port formed at one side of a bottom surface thereof and a central rotation shaft protruding downward from the center of the bottom surface, and
the lower body part has a rotation shaft receiving groove-forming part into which the central rotation shaft is inserted, and a supply port formed at one side of an upper surface thereof,
wherein the regulator-integrated drip chamber is configured to adjust the flow rate of the infusion fluid as the lower body part rotates around the central rotation shaft.

3. The regulator-integrated drip chamber of claim 2,
wherein an insertion groove is formed at a lower end of the central rotation shaft,
wherein the locking device comprises:
a locking body disposed between the central rotation shaft and the rotation shaft receiving groove-forming part, the locking body forming a lower end hole and internal threads on an inner surface thereof;
a central member disposed in the lower end hole, the central member including a locking end having external threads on an outer surface thereof that are screw-engaged with the internal threads; and
a tightening knob configured to rotate the central member,
wherein in a locking mode, an upper end of the central member is configured to be forcibly fitted into the insertion groove by the operation of the tightening knob to restrict rotation of the lower body part.

4. The regulator-integrated drip chamber of claim 2,
wherein threads are formed on an outer surface of a lower portion of the central rotation shaft, and
the rotation shaft receiving groove-forming part has a tubular shape with slits that are spaced apart in the circumferential direction,
wherein the central rotation shaft is inserted and coupled through the rotation shaft receiving groove-forming part such that the threads of the central rotation shaft are exposed at a lower side thereof,
wherein the locking device comprises:
a locking body screw-coupled to the lower portion of the central rotation shaft via internal threads of the locking body; and
a tightening knob connected to a lower end of the locking body and configured to rotate the locking body,
wherein in a locking mode, the tightening knob is configured to press the rotation shaft receiving groove-forming part to restrict the rotation of the lower body part.

5. The regulator-integrated drip chamber of claim 1, further comprising:
a flow rate sensor provided at one side of the body portion and configured to detect infusion droplets passing through the body portion and measure a supplied amount of infusion fluid;
a floating member configured to float on the infusion fluid stored in the body portion and close the discharge hole when the infusion supply is completed; and
a flow rate calculator configured to calculate a flow rate and a total supplied amount based on an interval between infusion droplets or the number of droplets supplied during a certain period, based on measurement data from the flow rate sensor,
wherein the flow rate sensor comprises at least one of an optical sensor, an infrared sensor, or a laser sensor to detect the supplied infusion droplets.

6. An infusion set including the regulator-integrated drip chamber according to any one of claims 1 to 5, the infusion set comprising:
the infusion bag;
the supply hose;
a catheter;
a flow control unit provided at one side of the supply hose and configured to regulate the flow rate of the infusion fluid supplied; and
a management server configured to calculate the flow rate and a total supplied amount of the infusion fluid based on an interval between infusion droplets or the number of droplets supplied during a certain period, using measurement data received from a flow rate sensor.

7. The infusion set of claim 6, further comprising:
a setting module configured to set a supplied amount and the flow rate of the infusion fluid;
a controller configured to control the flow control unit based on the measurement data and the preset flow rate and to stop the infusion when the total supplied amount reaches the preset supplied amount;
a liquid level sensor configured to measure a level of the infusion fluid in the drip chamber; and
a residual infusion fluid calculator configured to compute the remaining amount of infusion fluid from the drip chamber to a tip of the catheter based on the level of the infusion fluid,
wherein the controller is configured to control the flow control unit to stop the infusion when a value obtained by subtracting the remaining amount of infusion fluid from the total supplied amount reaches the preset supplied amount.
